# EUROPEAN PATENT APPLICATION

(11) **EP 3 498 698 A1**
(43) Date of publication of application: **19.06.2019**
(21) Application number: 18209083.7
(22) Date of filing: 29.11.2018
(51) Int. Cl.: C07D 257/04, A61P 9/12, A61K 31/41

(54) **SOLID FORMS OF VALSARTAN DISODIUM AND PROCESS OF PREPARATION THEREOF**

(30) Priority: 15.12.2017 IN 201711045149
(71) Applicant: MANKIND PHARMA LTD, New Delhi - 110020 (IN)
(72) Inventor: Chaturvedi, Vivek, 122050 IMT, Manesar, Gurugram (IN); Bhashkar, Bhuwan, 122050 IMT, Manesar, Gurugram (IN); Kumar, Anil, 122050 IMT, Manesar, Gurugram (IN)
(74) Representative: dompatent von Kreisler Selting Werner - Partnerschaft von Patent- und Rechtsanwälten mbB

(57) **Abstract**

The present invention relates to substantially pure solid forms of valsartan disodium and process of preparation thereof. Specifically the present invention relates to amorphous and crystalline form of valsartan disodium.

The present invention further provides stable solid form of valsartan disodium having moisture content in the range of 1-20% w/w.

## Description

### FIELD OF THE INVENTION

The present invention relates to substantially pure solid forms of valsartan disodium and process of preparation thereof. Specifically the present invention relates to amorphous and crystalline form of valsartan disodium.

The present invention further provides stable solid form of valsartan disodium having moisture content in the range of 1-20% w/w.

### BACKGROUND OF THE INVENTION

(S)-N-(1-Carboxy-2-methyl-prop-1-yl)-N-pentanoyl-N-[2'-(1H-tetrazol-5-yl)biphenyl-4-ylmethyl]-amine generically known as valsartan has the following structure:

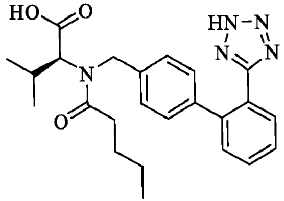

Valsartan is a non-peptide, orally active specific angiotensin II antagonist acting on the AT₁ receptor subtype. Valsartan is prescribed for the treatment of hypertension and is marketed as a free acid under the name trade name DIOVAN, however, its combination with diuretics, such as hydrochlorothiazide is also known as antihypertensive agent.

The active ingredient valsartan is the free acid which is described in EP 0443983. The synthesis of valsartan and its intermediates was reported in U.S. Pat. No. 5,399,578 and Bioorganic & Medicinal Chemistry Letters, vol. 4, page 29-34, 1994.

WO 2002/06253 discloses salts of valsartan along with their respective crystalline, partly crystalline and amorphous forms. The said patent application discloses preparation of valsartan disodium by dissolving valsartan in ethanol and mixing the solution so obtained with sodium hydroxide solution. The residue so obtained is stirred in hot acetonitrile and filtered to give white powder of valsartan disodium. It also discloses process for the preparation of crystalline form of valsartan disodium wherein said process includes addition of 2N sodium hydroxide solution to a solution of valsartan in isopropanol. Suspending amorphous residue of the disodium salt so obtained in isopropanol, then in acetone and water which is followed by addition of methyl tert-butyl ether. After stirring, cooling, filtering and washing with methyl tert-butyl ether, is obtained as a moist filter cake which is dried to get hygroscopic crystal powder.

WO 2003/066606 discloses various salts such as magnesium, sodium and calcium salts of valsartan along with their respective crystalline, partly crystalline and amorphous forms.

WO 2017/009784 provides a process for the preparation of amorphous valsartan disodium wherein said process includes dissolving valsartan in acetone and addition of aq. sodium hydroxide solution followed by concentrating the solution so obtained. Treated the residue thus obtained with methyl tert-butyl ether to get amorphous solid.

IN 201621010377 discloses amorphous form of valsartan disodium hydrate and process of preparation thereof.

Although crystalline and amorphous forms of valsartan (disodium) salts are known in the prior published references, however there is a need to provide a form of valsartan disodium having high purity and stability as compared to the known forms or polymorphs of valsartan disodium. There is a need for more stable and pure solid forms of valsartan disodium, which are easier to manage in the drying or grinding processes, and/ or the final stage of the chemical preparation process, and also in the steps for preparing the pharmaceutical formulations.

### OBJECT OF THE INVENTION

The main object of the present invention is to prepare substantially pure solid forms of valsartan disodium represented by Formula-A, wherein said solid forms are amorphous and crystalline in nature;

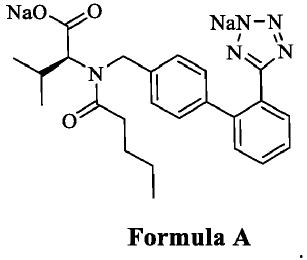

Another object of the present invention is to prepare a stable amorphous form of valsartan disodium having a moisture content of 3-8% w/w.

One another object of the present invention is to prepare a stable crystalline form of valsartan disodium having a moisture content of 11-16% w/w.

### SUMMARY OF THE INVENTION

The present invention relates to a substantially pure solid form of valsartan disodium and process of preparation thereof.

The present invention further relates to solid forms of valsartan disodium having moisture content of 1-20% w/w, wherein said solid form includes amorphous and crystalline form of valsartan disodium.

In one aspect, the present invention provides a substantially pure amorphous form of valsartan disodium with purity of 99% and above, wherein the total impurity is less than about 1% w/w.

In another aspect, the present invention provides a stable amorphous form of valsartan disodium characterized by Differential Scanning Calorimetry having isotherm peak ranging from 92.23°C to 121.15°C.

In another aspect the present invention provides a substantially pure amorphous form of valsartan disodium free of impurities of Formula I, II and III, wherein each impurity is ≤ 0.3% w/w; 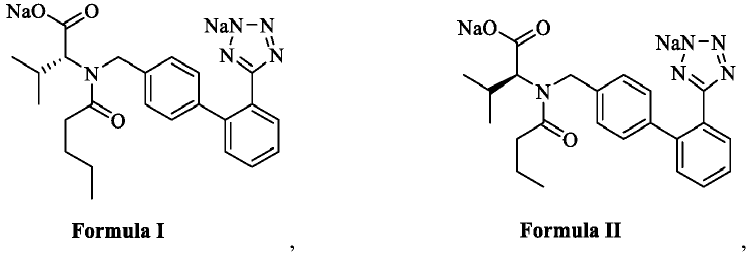 and

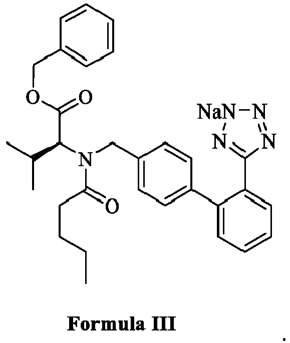

In another aspect, the present invention provides a stable and substantially pure amorphous form of valsartan disodium with moisture content of 3-8% w/w, wherein said amorphous form is free of impurities of Formula I, II and III; 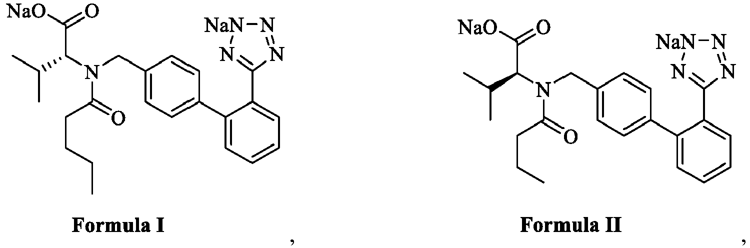 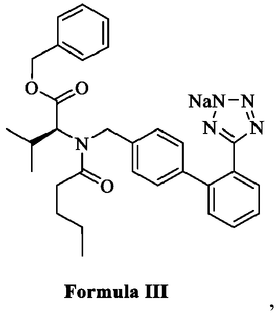 wherein each impurity is ≤ 0.3% w/w.

In another aspect, the present invention provides a process for preparation of amorphous form of valsartan disodium, the process comprising the steps of:
(a) treating valsartan with a sodium ion source in suitable first solvent(s);
(b) optionally distilling the first solvent(s),
(c) adding suitable second solvent(s) and stirring for a time effective to form solid mass and filtering the solid mass;
(d) optionally washing the solid mass of step (c) in suitable third solvent(s); and
(e) isolating amorphous form of valsartan disodium.

In another aspect, the present invention provides a process for preparation of amorphous form of valsartan disodium, wherein said process comprising the steps of:
(a) providing solution of valsartan in methanol;
(b) providing solution of sodium hydroxide in methanol and heated at a temperature in the range of 40-70°C;
(c) adding solution of sodium hydroxide as obtained in step (b) to solution of valsartan as obtained in step (a) and stirring to get a solution;
(d) cooling to a temperature below 30°C and adding n-heptane and stirring for a time effective to form solid mass;
(e) filtering the solid mass and washing with n-heptane; and
(f) drying to get amorphous form of valsartan disodium.

### DETAILED DESCRIPTION OF THE INVENTION

### Description of drawings

Fig. 1 represents X-Ray Powder Diffraction Pattern of amorphous form of valsartan disodium

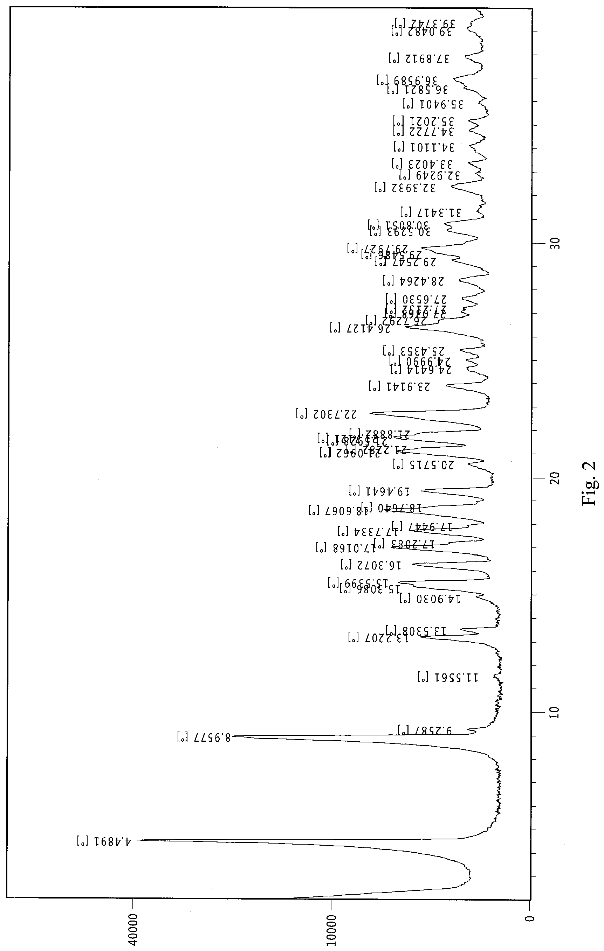

Fig. 2 represents X-Ray Powder Diffraction Pattern of crystalline form of valsartan disodium
Fig. 3 represents Differential Scanning Calorimetry of amorphous form of valsartan disodium

### Definitions:

The term "substantially pure" as referred in the context of the present invention relates to substance that has purity preferably between about 95% and 100% by HPLC and total impurities below 2% w/w, more preferably between about 99% and 100% of purity by HPLC and below 1%w/w of the total impurities, and, most preferably, between about 99.5% and 100% of purity by HPLC and below 0.25% w/w of total impurities.

It must be noted that, as used in the specification and the appended claims, the singular forms "a," "an" and "the" include plural referents unless the context clearly dictates otherwise. Thus, for example, reference to "a solvent" includes mixtures of solvents.

"Optional" or "optionally" means that the subsequently described event or circumstance may or may not occur, and that the description includes instances where said event or circumstance occurs and instances where it does not.

The present invention will now be explained in details. While the invention is susceptible to various modifications and alternative forms, specific embodiment thereof will be described in detail below. It should be understood, however that it is not intended to limit the invention to the particular forms disclosed, but on the contrary, the invention is to cover all modifications, equivalents, and alternative falling within the scope of the invention as defined by the appended claims.

The steps of a method may be providing more details that are pertinent to understanding the embodiments of the present invention and so as not to obscure the disclosure with details that will be readily apparent to those of ordinary skill in the art having benefit of the description herein.

Further characteristics and advantages of the process according to the invention will result from the description herein below of preferred exemplary embodiments, which are given as indicative and non-limiting examples.

Accordingly, the present invention provides a substantially pure solid form of valsartan disodium and process of preparation thereof.

In another embodiment, the present invention provides a solid form of valsartan disodium having moisture content of 1-20% w/w, wherein said solid form is selected from amorphous form, and crystalline form.

In another embodiment, the present invention provides a substantially pure amorphous form of valsartan disodium with purity of 99% and more, wherein the total impurity is less than about 1% w/w.

In a preferred embodiment, the present invention provides a substantially pure amorphous form of valsartan disodium free of impurities of Formula I, II and III, wherein each impurity is ≤ 0.3% w/w; 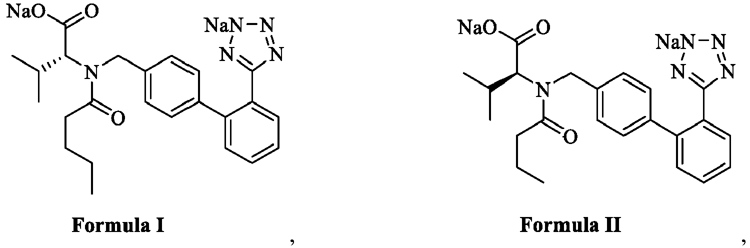 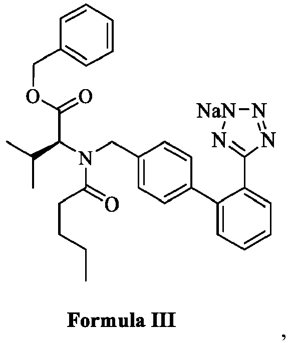 wherein said amorphous form is characterized by Differential Scanning Calorimetry having isotherm peak at about 92.23°C to 121.15°C.

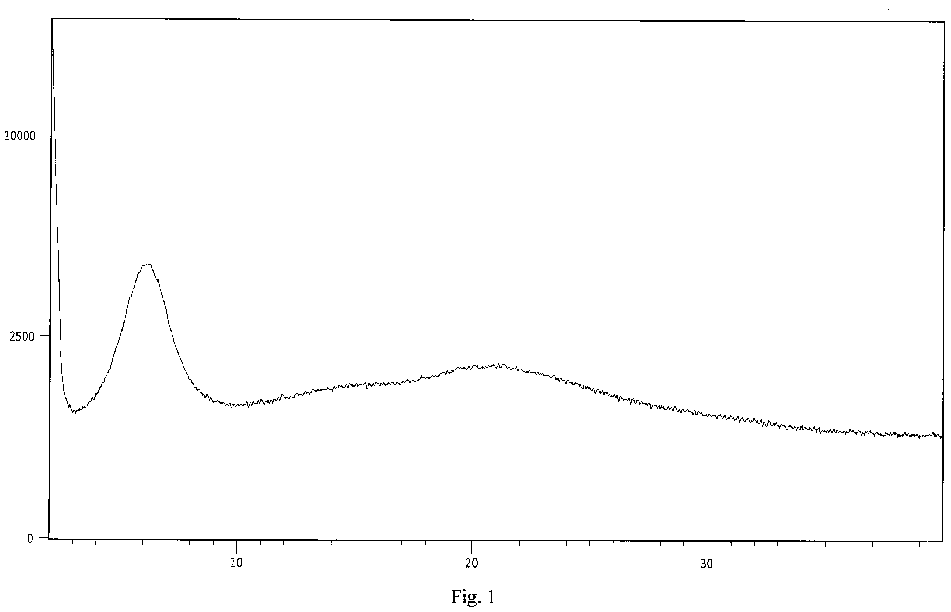

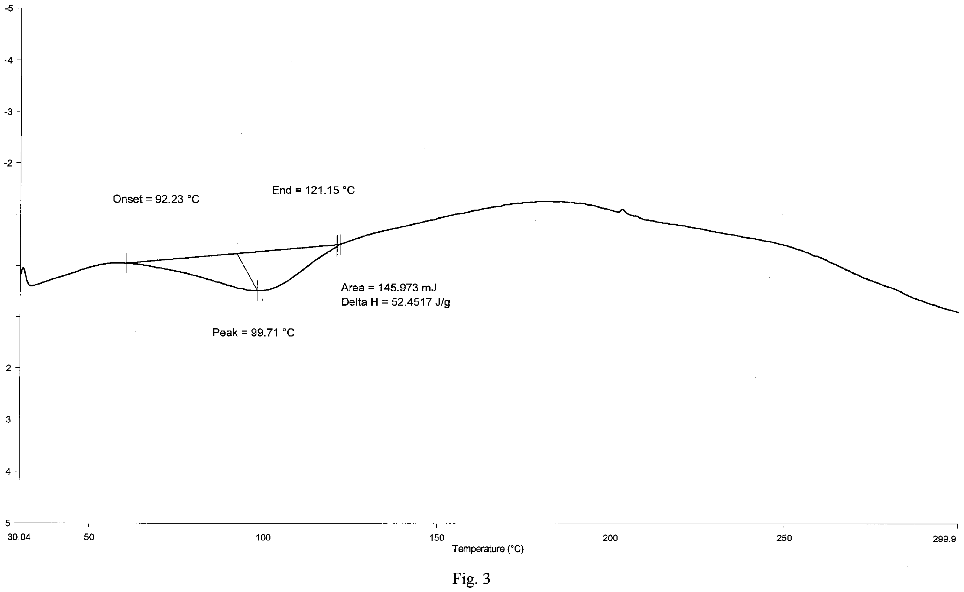

In another embodiment, the present invention provides a stable and substantially pure amorphous form of valsartan disodium with moisture content of 3-8% w/w wherein said amorphous form is characterized by X-Ray powder diffraction pattern as represented in Fig. 1, and differential scanning calorimetry having isotherm peak ranging from 92.23°C to 121.15°C as represented in Fig. 3.

In another embodiment, the present invention provides a stable and substantially pure amorphous form of valsartan disodium with moisture content of 3-8% w/w, wherein said amorphous form is free of impurities of Formula I, II and III; 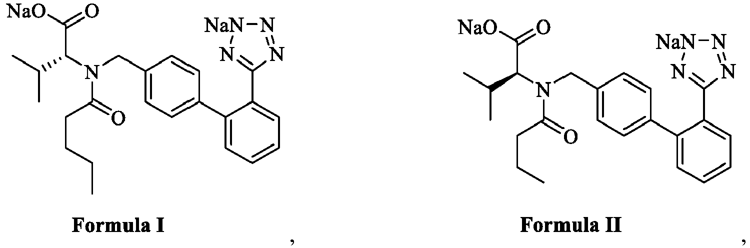 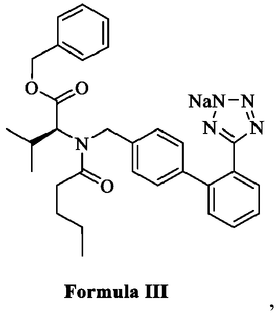 wherein each impurity is ≤ 0.3% w/w.

In another embodiment, the present invention provides a process for preparation of amorphous form of valsartan disodium, wherein said process comprising the steps of:
(a) treating valsartan with a sodium ion source in suitable first solvent(s);
(b) optionally distilling the first solvent(s),
(c) adding suitable second solvent(s) and stirring for a time effective to form solid mass and filtering the solid mass;
(d) optionally washing the solid mass of step (c) in suitable third solvent(s); and
(e) isolating amorphous form of valsartan disodium.

In a preferred embodiment, the sodium ion source is selected from sodium hydroxide, sodium carbonate, sodium bicarbonate, sodium methoxide or mixture thereof.

In another preferred embodiment, the sodium ion source is added to the reaction solution either in the solid form or in form of solution in water or organic solvent(s) wherein said organic solvent(s) is selected from ethanol, methanol, isopropanol, butanol, tert-butanol, iso-butanol and mixture thereof.

In another embodiment, the solvent(s) used in preparation of amorphous form of valsartan disodium is selected from the group comprising of water, methanol, ethanol, isopropanol, butanol, tert-butanol, iso-butanol, chloroform, dichloromethane, carbon tetrachloride, ethyl acetate, butyl acetate, isopropyl acetate, isopropenyl acetate, tetrahydrofuran, methyl tetrahydrofuran, dioxane, methyl tert-butyl ether, isopropyl ether, isobutyl ether, diethyl ether, acetone, methyl ethyl ketone, methyl isobutyl ketone, hexane, cyclohexane, heptane, and mixture thereof.

In a preferred embodiment, the valsartan is reacted with sodium ion source in presence of a solvent (i.e. first solvent) selected from the group comprising of water, methanol, ethanol, iso-propanol, butanol, iso-butanol, tert-butanol, acetone, methyl ethyl ketone, methyl isobutyl ketone, and mixture thereof.

In another preferred embodiment, the second solvent (s) as used in step (c) above is selected from the group comprising of methanol, ethanol, iso-propanol, butanol, iso-butanol, tert-butanol, acetone, methyl ethyl ketone, methyl isobutyl ketone, methyl tert-butyl ether, isobutyl ether, isopropyl ether, hexane, cyclohexane, heptane, tetrahydrofuran and mixture thereof.

In another preferred embodiment, the third solvent (s) as used in step (d) above is selected from the group comprising of methyl tert-butyl ether, isobutyl ether, isopropyl ether, hexane, cyclohexane, heptane, tetrahydrofuran, and mixture thereof.

In one another embodiment, the amorphous form of valsartan disodium is isolated by one or more methods such as distillation, evaporation, spray drying, freeze-drying, lyophilization, filtration under vacuum, decantation and centrifugation.

The amorphous form of valsartan disodium can be prepared by lyophilizing or spray drying the solution resulting from the addition of solvent during further purification and/ or the solution resulting from the addition of sodium ion source to valsartan in a suitable solvent.

In one another embodiment, the valsartan disodium is treated with sodium ion source in a ketonic solvent followed by stirring at ambient temperature for 1-2hr. The ketonic solvent is then distilled to give a solid material which is again treated with a ketonic solvent. The process is repeated twice to give a solid material which is then treated with a suitable hydrocarbon solvent such as n-heptane or cyclohexane at ambient temperature and stirred the suspension so obtained for 1-2 hr. Filtration is performed to get a solid mass which is washed with the suitable hydrocarbon solvent such as n-heptane or cyclohexane and then dried to give substantially pure amorphous form of valsartan disodium.

In another embodiment, the present invention provides a process for preparation of amorphous form of valsartan disodium, wherein said process comprising the steps of:
(a) providing solution of valsartan in methanol;
(b) providing solution of sodium hydroxide in methanol and heated at a temperature in the range of 40-70°C;
(c) adding solution of sodium hydroxide as obtained in step (b) to solution of valsartan as obtained in step (a) and stirring to get a solution;
(d) cooling to a temperature below 30°C and adding n-heptane and stirring for a time effective to form solid mass;
(e) filtering the solid mass and washing with n-heptane; and
(f) drying to get amorphous form of valsartan disodium.

Further, the present invention has the advantage of providing an amorphous form of valsartan disodium which is stable and has purity of 99.0% and above.

In another embodiment, the present invention provides a substantially pure amorphous form of valsartan disodium substantially free of any crystalline form.

In a preferred embodiment, the amorphous form of valsartan disodium of present application is stable on storage as shown in Table-1 which represents the data related to water content and impurity profile of stable amorphous form as measured by Karl Fischer method.

**Table 1:**

| | | | | | | | |
|---|---|---|---|---|---|---|---|
| 60%±5% (RH)25°C±2°C, | | Initial | 1^{st} month | 2^{nd} month | 3^{rd} month | 6^{th} month | Spec. Limit |
| | Water content (wt %) | 5.1 | 5.1 | 5.2 | 5.1 | 5.1 | NMT 7.0 |
| | Related compound B | 0.03 | 0.02 | 0.03 | 0.03 | 0.03 | NMT 0.20 |
| | Related compound C | ND | ND | ND | ND | ND | NMT 0.10 |
| | Related compound A | 0.29 | 0.28 | 0.29 | 0.29 | 0.29 | NMT 1.0 |
| | Any individual impurity | 0.04 | 0.05 | 0.05 | 0.05 | 0.05 | NMT 0.10 |
| 40°C±2°C, 75%±5% (RH) | Water content (wt %) | 5.1 | 5.2 | 5.2 | 5.1 | 5.1 | NMT 7.0 |
| | Related compound B | 0.03 | 0.01 | 0.01 | 0.01 | 0.01 | NMT 0.20 |
| | Related compound C | ND | ND | ND | ND | ND | NMT 0.10 |
| | Related compound A | 0.29 | 0.29 | 0.28 | 0.28 | 0.28 | NMT 1.0 |
| | Any individual impurity | 0.04 | 0.04 | 0.04 | 0.04 | 0.04 | NMT 0.10 |
| 2-8°C | Water content (wt %) | 5.1 | 5.20 | 5.15 | 5.20 | 5.18 | NMT 7.0 |
| | Related compound B | 0.03 | 0.03 | 0.03 | 0.03 | 0.03 | NMT 0.20 |
| | Related compound C | ND | ND | ND | ND | ND | NMT 0.10 |
| | Related compound A | 0.29 | 0.28 | 0.28 | 0.28 | 0.28 | Nmt 1.0 |
| | Any individual impurity | 0.04 | 0.03 | 0.03 | 0.03 | 0.03 | NMT 0.10 |

In another embodiment, the present invention is directed towards a pharmaceutical composition comprising a pharmaceutically effective amount of substantially pure amorphous form of valsartan disodium in combination with at least one pharmaceutically acceptable excipients and optionally with another pharmaceutical acceptable ingredient.

In another embodiment, the present invention provides a stable and substantially pure crystalline form of valsartan disodium with purity of 99% and more, wherein the total impurity is less than about 1% w/w.

In other embodiment, the present invention provides a substantially pure and stable crystalline form of valsartan disodium free of impurities of Formula I, II and III, wherein each impurity is ≤ 0.3% w/w.

In other embodiment, the crystalline form of valsartan disodium is characterized by XRPD peaks at about 4.48, 8.95, 9.25, 15.53, 17.01, 18.60, and 22.73 ±0.2°2θ.

In another embodiment, the crystalline form of valsartan disodium is characterized by XRPD peaks at about 4.48, 8.95, 9.25, 11.55, 13.22, 13.53, 14.90, 15.30, 15.53, 16.30, 17.01, 17.20, 17.73, 17.94, 18.60, 18.76, 19.46, 20.57, 21.09, 21.22, 21.59, 21.71, 21.88, 22.73, 23.91, 24.64, 24.99, 25.43, 26.41, 26.72, 27.03, 27.21, 27.65, 28.42, 29.25, 29.54, 29.79, 30.52, 30.80, 31.34, 32.39, 32.92, 33.40, 34.11, 34.77, 35.20, 35.94, 36.58, 36.95, 37.89, 39.04, and 39.37 ±0.2°2θ.

In another embodiment, the crystalline form of valsartan disodium is characterized by Differential Scanning Calorimetry having endotherm peak at about 234.98°C.

In one another embodiment, the present invention provides a crystalline form of valsartan disodium having moisture content of 11-16% w/w.

In one another embodiment, the present invention provides a process for preparation of crystalline form of valsartan disodium, the process comprising of:
(a) combining valsartan with a sodium ion source in a solvent,
(b) distilling and adding second solvent (s) and stirring for a time effective to form solid mass;
(c) optionally distilling and adding third solvent (s) and stirring for a time effective to form solid mass; and
(d) filtering and drying to give a crystalline form of valsartan disodium.

In a preferred embodiment, the sodium ion source is selected from sodium hydroxide, sodium carbonate, sodium bicarbonate, sodium methoxide or mixture thereof. The sodium ion source is added to the reaction solution either in the solid form or in form of solution in water or organic solvent.

In another embodiment, the solvents used in preparation of crystalline form of valsartan disodium is selected from the group comprising of water, methanol, ethanol, isopropanol, butanol, tert-butanol, iso-butanol, chloroform, dichloromethane, carbon tetrachloride, ethyl acetate, butyl acetate, isopropyl acetate, isopropenyl acetate, tetrahydrofuran, methyl tetrahydrofuran, dioxane, methyl tert-butyl ether, isopropyl ether, isobutyl ether, diethyl ether, acetone, methyl ethyl ketone, methyl isobutyl ketone, hexane, cyclohexane, heptane, and mixture thereof.

In a preferred embodiment, the valsartan is reacted with sodium ion source in presence of a solvent selected from the group comprising of isopropyl alcohol, methanol, ethanol, isobutanol, butanol, tert-butanol and mixture thereof.

In another preferred embodiment, after distillation of solvents in step (b), a crude mass is obtained to which may be added a second solvent selected from the group comprising of water, acetone, methyl ethyl ketone, methyl isobutyl ketone, methyl tert-butyl ether, isopropyl ether, isobutyl ether, tetrahydrofuran, hexane, cyclohexane, heptane, and mixture thereof.

The filtration and drying of crystalline form of valsartan disodium may be performed by filtration under vacuum, simple filtration, spray drying, drying under reduced pressure, lyophilization, and drying under heating in oven.

In another embodiment, the present invention provides crystalline form of valsartan disodium having purity of 99.0% and above.

Further, the present invention has the advantage of providing a highly crystalline form of valsartan disodium that has more stability compared to known forms of valsartan.

In another embodiment, the present invention is directed towards a pharmaceutical composition comprising a pharmaceutically effective amount of substantially pure crystalline form of valsartan disodium in combination with at least one pharmaceutically acceptable excipients.

In another embodiment, the present invention provides solid dispersion of valsartan disodium with pharmaceutical acceptable carrier.

In another embodiment, the present invention provides solid dispersion of valsartan disodium with pharmaceutical acceptable carrier; wherein said solid dispersion is having moisture content of 1-20% w/w.

In a preferred embodiment, the said pharmaceutical acceptable carrier used for preparing the solid dispersion is selected from polymers and copolymers. Preferably, said pharmaceutical acceptable carrier is selected from hydroxypropylmethyl cellulose, hydroxypropyl cellulose, povidone, polyethylene glycol, ethylcellulose, liposomes, methacrylic acid copolymer, polyvinyl acetate, carboxymethyl ethyl cellulose, carboxymethyl cellulose phthalate, hydroxypropylmethyl cellulose phthalate, hydroxypropylmethyl cellulose acetate succinate, poly month purpose acrylic resin, carbopol, alginate, carrageenan, carboxy lactone acetate, polyvinyl alcohol, pregelatinized starch, crosslinked starch, sodium carboxymethyl starch, dextrin, polyethylene oxide, chitosan, chitosan, ion exchange resin and at least one of collagen.

In other embodiment, the present invention provides a solid dispersion comprising of substantially pure valsartan disodium and pharmaceutical acceptable carrier, wherein said substantially pure valsartan disodium is free of impurities of Formula I, II and III, wherein each impurity is ≤ 0.3% w/w.

In a preferred embodiment, the valsartan disodium is characterized by the particle size distribution wherein, d₉₀ is between 0.1µm to 200µm, wherein valsartan disodium is isolated as amorphous and/or crystalline form.

In a preferred embodiment, the valsartan disodium is characterized by particle size distribution wherein, d₉₀ is between 2.0 µm to 150µm, wherein valsartan disodium is isolated as amorphous and/or crystalline form.

In another embodiment, the present invention is directed toward a method for treating hypertension or elevated blood pressure in a patient comprising administering a pharmaceutical composition comprising a pharmaceutically effective amount of the substantially pure amorphous form of valsartan disodium to a patient in need thereof.

Certain specific aspects and embodiments of the present application will be explained in greater detail with reference of the following examples, which are provided only for purposes of illustration and should not able constructed as limited the scope of the application in any manner.

### EXAMPLES:

### Experiment 1: Amorphous form of Valsartan disodium

Process 1: Charged 2.03 eq of sodium hydroxide to the 2 vol of methanol and stirred at 55-60°C for 20 to 30 min, then cooled to 25-30°C and added drop wise to the 3 vol of methanol and 25g of valsartan solution. Stirred the solution thus obtained at 25-30°C for 1 hr and followed by degassing for 1-2 hr at 50-60°C. Cooled to 25-30°C and charged 5V of n-heptane and stirred for 1-2 hr at RT. Filtered under nitrogen and washed with 1 vol of n heptane and dried under reduced pressure at 60-65°C to get 26.5g of amorphous form of valsartan disodium with purity of 99.91% by HPLC.
Moisture Content: 3.10%

### Impurities:

Formula I: 0.28%
Formula III: ND
Formula II: 0.04%

Process 2: Charged isopropyl alcohol (10.0V) to 10.0g of valsartan and added 2.0 mole equivalent of sodium hydroxide solution. Stirred for 1.0hr at room temperature and after completion of reaction, distilled out the solvent at 50-55°C. Added 10.0V of isopropyl ether or 10.0V of methyl tert-butyl ether at room temperature and stirred at room temperature. Filtered and dried the solid at 55-60°C under vacuum to get 10.9 g of amorphous form of valsartan disodium.
Purity: 99.92%

Process 3: Charged 2 vol of ethanol to 50.0g of valsartan and added sodium ethoxide (prepared in 3 vol of ethanol and 2.03 eq of sodium hydroxide) solution and stirred at 65-70°C for 20 to 30 min, then cooled to 25-30°C and added drop wise to the 2 vol of ethanol followed by stirring at 25-30°C for 1 hr. Distilled off 3.5 vol of ethanol at 40-45°C under reduced pressure and cooled to 25 to 30°C followed by drop wise addition to the 20 vol of MTBE or IPE having 0-5°C temperature. Stirred at 0-5°C for 1-2 hr and filtered under nitrogen and washed with 1 vol of MTBE or IPE. Dried the solid so obtained under reduced pressure at 60-65°C to get 42.50g of amorphous form of valsartan disodium.
Purity: 99.9%

Process 4: Charged sodium hydroxide solution (2.03 eq of sodium hydroxide in 1.5 vol of DM water) to the 50.0g of valsartan in 5 vol of acetone and stirred at 25-30°C for 1 Hr. Distilled the solvents under reduced pressure at 65-70°C and added 5 vol of acetone and distilled atmospherically and again added 5 vol of acetone and distilled atmospherically. Degassed for 2hr at 65-70°C then cooled to 25-30°C and added 5 vol of n-heptane/ cyclohexane and stirred at 25-30°C for 1-2 hr and then filtered under nitrogen and washed with 1 vol of n-heptane /cyclohexane and dried under reduced pressure at 60-65°C to get 53.0 gms of amorphous form of valsartan disodium.
Yield: 99.71%

Process 5: Charged sodium hydroxide solution (2.03 eq of sodium hydroxide in 1.5 vol of DM water) to the 50.0g of valsartan in 5 vol of acetone, stirred at 25-30°C for 1 hr, then distilled under reduced pressure at 60-65°C. Added 5 vol of acetone and distilled under reduced pressure at 60-65°C and again added 5 vol of acetone and distilled under reduced pressure at 60-65°C. Degassed for 2hr at 60-65°C then added 3 vol of acetone and stirred to get cleared solution at 45-50°C, cooled to 0-5°C and slowly added 500 ml of cyclohexane, stirred for 1-2 hr at 0-5°C. Filtered and washed with 1 vol of cyclohexane, dried under reduced pressure at 60 -65°C to get 52.0g of amorphous form of valsartan disodium.
Yield: 99.92%

### Experiment 2: Crystalline form of Valsartan disodium

Process 1: Charged isopropyl alcohol (5.0V), valsartan free acid (50g) and added sodium hydroxide solution (2.0 mole equivalent) (IV) at RT. Stirred for 1.0-2.0hr at room temperature and after completion of reaction, distilled out the solvent at 50-55°C. Degassed completely, added 10.0V of methyl tert-butyl ether at room temperature and stirred 2-4hrs at room temperature. Filtered and dried the solid at 55-60°C under vacuum to get 56.0g crystalline form of Valsartan disodium.
Purity: 99.80%
Moisture content: 11.07%

Process 2: Charged 9.3V of isopropyl alcohol to 200.0g of valsartan free acid and added 2.3V of 2.0N of sodium hydroxide solution. Stirred the solution at room temperature for 1.0 hr. Distilled the solvent at 40-45°C and added 4.5V of isopropyl alcohol followed by distillation under vacuum at 40-45°C. Charged 3.5V of acetone and 0.09V of water to get clear solution and added 9.3V of methyl tert-butyl ether and stirred overnight at room temperature. Filtered the solid and washed with 1 volume of methyl tert-butyl ether. Dried the solid at 55 to 60°C to get 240.0g of crystalline form of valsartan disodium.
Moisture content: 13.14%

### Impurities:

Formula I: 0.3%
Formula II: 0.01%
Formula III: ND

### Experiment 3: Solid dispersion of valsartan disodium with HPMC (hydroxypropyl methyl cellulose):

Process 1: 1g of valsartan disodium and 2g of hydroxypropyl methyl cellulose were dissolved in water at 60°C and stirred the solution for 1-2h. The clear solution was taken Buchi rotavapour and evaporated under reduced pressure to obtain the solid dispersion of valsartan disodium with HPMC.

Process 2: 1g of valsartan disodium and 5g of hydroxypropyl methyl cellulose were dissolved in water and methanol (1:1) at 40°C and stirred the solution for 1-2h. The clear solution was taken Buchi rotavapour and evaporated under reduced pressure to obtain the solid dispersion of valsartan disodium with HPMC.

Process 3: 1g of valsartan disodium and 7g of hydroxypropyl methyl cellulose were dissolved in methanol at 40°C and stirred the solution for 1-2h. The clear solution was taken Buchi rotavapour and evaporated under reduced pressure to obtain the solid dispersion of valsartan disodium with HPMC.

## Claims

1. A stable and substantially pure amorphous form of valsartan disodium free of impurities of Formula I, II and III, wherein each impurity is ≤ 0.3% w/w; 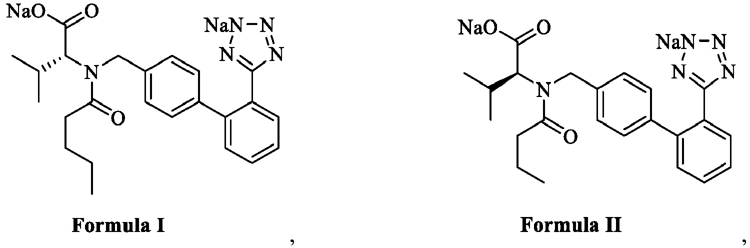 and

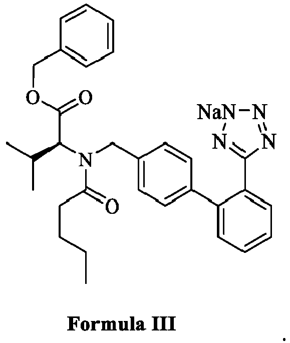

2. The amorphous form of valsartan disodium as claimed in claim 1, wherein said amorphous form is **characterized by** Differential Scanning Calorimetry having isotherm peak ranging from 92.23°C to 121.15°C.

3. The amorphous form of valsartan disodium as claimed in claim 1, wherein said amorphous form is **characterized by** moisture content of 3-8% w/w.

4. A process for preparation of amorphous form of valsartan disodium, wherein said process comprising of:
(a) treating valsartan with a sodium ion source in suitable first solvent(s);
(b) optionally distilling the first solvent(s),
(c) adding suitable second solvent(s) and stirring for a time effective to form solid mass and filtering the solid mass;
(d) optionally washing the solid mass of step (c) in suitable third solvent(s); and
(e) isolating amorphous form of valsartan disodium.

5. The process as claimed in claim 4, wherein said first solvent is selected from the group comprising of water, methanol, ethanol, iso-propanol, butanol, iso-butanol, tert-butanol, acetone, methyl ethyl ketone, methyl isobutyl ketone, and mixture thereof; and wherein said second solvent is selected from the group comprising of methanol, ethanol, iso-propanol, butanol, iso-butanol, tert-butanol, acetone, methyl ethyl ketone, methyl isobutyl ketone, methyl tert-butyl ether, isobutyl ether, isopropyl ether, hexane, cyclohexane, heptane, tetrahydrofuran, and mixture thereof;
and wherein said third solvent is selected from the group comprising of methyl tert-butyl ether, isobutyl ether, isopropyl ether, hexane, cyclohexane, heptane, tetrahydrofuran, and mixture thereof.

6. The process as claimed in claim 4, wherein said sodium ion source is selected from sodium hydroxide, sodium methoxide, sodium carbonate, sodium bicarbonate or mixture thereof.

7. The process as claimed in claim 4, wherein said amorphous form of valsartan disodium is isolated by one or more of distillation, evaporation, spray drying, freeze-drying, lyophilization, filtration under vacuum, decantation and centrifugation.

8. A process for preparation of amorphous form of valsartan disodium, wherein said process comprising the steps of:
(a) providing solution of valsartan in methanol;
(b) providing solution of sodium hydroxide in methanol and heated at a temperature in the range of 40-70°C;
(c) adding solution of sodium hydroxide as obtained in step (b) to solution of valsartan as obtained in step (a) and stirring to get a solution;
(d) cooling to a temperature below 30°C and adding n-heptane and stirring for a time effective to form solid mass;
(e) filtering the solid mass and washing with n-heptane; and
(f) drying to get amorphous form of valsartan disodium.

9. The process as claimed in claim 8, wherein said amorphous form of valsartan disodium is isolated with moisture content of 3-8% w/w.

10. A composition comprising substantially pure amorphous form of valsartan disodium as claimed in claim 1, along with at least one pharmaceutical acceptable excipients wherein said composition optionally comprises of another active pharmaceutical ingredient.
